# EUROPEAN PATENT SPECIFICATION

(11) **EP 4 245 357 B1**
(45) Date of publication and mention of the grant of the patent: **25.12.2024**
(21) Application number: 23189471.8
(22) Date of filing: 23.10.2019
(51) Int. Cl.: A61N 1/36, A61N 1/04, A61N 1/05

(54) **CURRENT SOURCE FOR NEUROSTIMULATION**
STROMQUELLE ZUR NEUROSTIMULATION
SOURCE DE COURANT POUR NEUROSTIMULATION

(30) Priority: 23.10.2018 AU 2018904014
(43) Date of publication of application: 20.09.2023
(62) Divisional of application: 19875139.8
(73) Proprietor: Saluda Medical Pty Ltd, Macquarie Park, NSW 2113 (AU)
(72) Inventor: SINGLE, Peter Scott Vallack, Artarmon, 2064 (AU)
(74) Representative: Abel & Imray LLP

(56) References cited:
- WO-A1-2017/142948
- WO-A1-2018/080753
- US-A1- 2017 157 410
- US-A1- 2017 173 326
- US-A1- 2018 071 513

## Description

### Technical Field

The present invention relates to a current source for a current controlled implantable stimulator such as a neurostimulator, and in particular to a current source configured to deliver current pulses simultaneously to more than one electrode.

### Background of the Invention

Electrical neuromodulation is used or envisaged for use to treat a variety of disorders including chronic pain, Parkinson's disease, and migraine, and to restore function such as hearing and motor function. A neuromodulation system applies an electrical pulse to neural tissue in order to generate a therapeutic effect. Such a system typically comprises an implanted electrical pulse generator, and a power source such as a battery that may be rechargeable by transcutaneous inductive transfer. An electrode array is connected to the pulse generator, and is positioned close to the neural pathway(s) of interest. An electrical pulse applied to the neural tissue by an electrode causes the depolarisation of neurons, which generates propagating action potentials whether antidromic, orthodromic, or both, to achieve the therapeutic effect.

When used to relieve chronic pain for example, the electrical pulse is applied to the dorsal column (DC) of the spinal cord and the electrode array is positioned in the dorsal epidural space. The dorsal column fibres being stimulated in this way inhibit the transmission of pain from that segment in the spinal cord to the brain.

In general, the electrical stimulus generated in a neuromodulation system triggers a neural action potential which then has either an inhibitory or excitatory effect. Inhibitory effects can be used to modulate an undesired process such as the transmission of pain, or excitatory effects can be used to cause a desired effect such as the contraction of a muscle or stimulation of a sensory nerve such as the auditory nerve.

Accurately delivering stimuli to a desired location upon a nerve is a key factor in the therapeutic benefit perceived by the implant recipient. For example, some approaches to spinal cord stimulation (SCS) for treating chronic pain seek to induce paraesthesia which covers, or is co-located with, a dermatome or body region associated with the condition being treated. To this end, both the surgical implantation procedure and the post-surgical device fitting process involve considerable efforts to optimise very precisely a location on the spinal cord at which the therapy is to be delivered by the implanted electrode array. Such efforts are however complicated by the physical size of the implanted lead or electrode array. Typical electrode arrays have electrodes which are a number of millimetres wide, and typical inter-electrode spacings are several millimetres or more, for example SCS percutaneous leads commonly have electrodes which are 3 mm wide and which have an inter-electrode spacing of 4 mm, so that the electrode centres are 7 mm apart. Relative to the much smaller differences between positions of nodes of Ranvier in a fibre population of interest, and the small internodal spacing of nodes of Ranvier on each such axon, the large electrode size and large electrode spacings of typical neuromodulation leads provide for relatively poor spatial control over the location at which stimuli can be delivered.

To address this problem, "current steering" or a "virtual electrode" seeks to effectively interpose stimuli delivery to locations between the physical electrodes, by using more than one active stimulus electrode to deliver contemporaneous stimuli which together give the effect of delivering a single stimulus at an apparent location between the electrodes, with the apparent location being defined by, and thus controllable by, the relative magnitude of the contemporaneous stimuli. However, this approach requires multiple duplications of the required hardware for stimulation, such as duplicate current sources and duplicate current control circuitry. Current steering also considerably complicates clinical fitting of the device due to the significant increase in the number of control parameters which must be clinically defined. Current steering also complicates ongoing control of the therapy such as when the implant recipient wishes to adjust the stimulation intensity when making postural changes. US 2018/071513 discloses an example of a method of controlling electrical stimulation therapy, with a periodical adjustment to prevent action potentials in the tissue of a patient. Technological background may be provided by WO 2018/080753, WO 2017/142948, US 2017/157410, and US 2017/173326

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

In this specification, a statement that an element may be "at least one of" a list of options is to be understood that the element may be any one of the listed options, or may be any combination of two or more of the listed options.

### Summary of the Invention

The invention is defined in the appended claims. Aspects, embodiments and examples disclosed herein which do not fall within the scope of the appended claims do not form part of the invention, and are merely provided for illustrative purposes.

According to an example which is not covered by the subject matter of the claims, there is an implantable neurostimulator comprising:
an implantable electrode array comprising a plurality of stimulus electrodes, each stimulus electrode configured to deliver electrical stimuli to neural tissue;
an implantable control module configured to produce the electrical stimuli delivered by the stimulus electrodes, the control module configured to effect current steering and comprising a plurality of related current sources, each current source configured to deliver a respective stimulus current which is defined in a first part by a shared current control signal which is shared by each of the related current sources, and which is defined in a second part by a respective unique current control signal which is not shared by all of the related current sources.

According to a second example which is not covered by the subject matter of the claims, there is a method of current steering, comprising:
generating a plurality of contemporaneous stimuli by controlling a respective plurality of current sources, each current source configured to deliver a respective stimulus current which is defined in a first part by a shared current control signal which is shared by each of the related current sources, and which is defined in a second part by a respective unique current control signal which is not shared by all of the related current sources; and
delivering the contemporaneous stimuli to neural tissue via an implantable electrode array comprising a plurality of stimulus electrodes.

According to a further example which is not covered by the subject matter of the claims, there is a computing device configured to carry out the method of the second example. According to a further example there is a non-transitory computer readable medium for current steering, comprising instructions which, when executed by one or more processors, causes performance of the method of the second example.

The present examples provide for the plurality of current sources to have a portion of their operation in common, as defined and controlled by the shared current control signal. The present examples recognises that current steering can be beneficially addressed by utilising both a shared current control signal and respective unique current control signals. The shared current control signal can then be used as a single control parameter to effect adjustments in net stimulation intensity, as may be required when the implant recipient changes posture. This is particularly important in examples which measure the evoked neural response and use such measurements for automated feedback control of the stimulation intensity, as the feedback loop speed is minimally hampered by a single intensity control parameter and a higher stimulation rate and/or feedback rate can thus be supported.

In some examples, a majority of the operation of each current source is controlled by the shared current control signal. For example, for digitally controlled current sources, a majority of a set of control bits used to control each current source may be defined by the shared current control signal. In some such examples, at least the most significant half of the control bits are defined by the shared current control signal, with the remaining least significant bits being defined by the respective unique current control signal.

In one example, the 12 most significant bits of a 16 bit current source are defined by the shared current control signal, and the remaining 4 least significant bits are defined by the respective unique current control signal. Such examples recognise that for 16 bit control providing 4, or about 4, of the least most significant bits of control to the respective unique current control signal permits sufficiently fine spatial resolution for current steering, so that the virtual electrode can be selectively positioned at a large number of locations between the active stimulus electrodes in use. Moreover, such examples recognise that providing 12, or so, bits of control to the shared current control signal provides for a suitably large control range of net stimulation intensity, as is typically required in the SCS field in particular due to the large effect that electrode to nerve distance, and patient movement, can have on recruitment.

In some examples, four or more related current sources are provided. Such examples are particularly advantageous in permitting the use of current steering to position an apparent location of stimulation between electrodes in both a caudorostral direction and also between electrodes in a mediolateral direction, for example when a paddle lead with electrodes distributed in a two dimensional array is in use, or when two adjacent linear (one dimensional) array leads are in use.

In some examples current sources can be respectively assigned to each respective stimulus electrode and may have 4-bit control over their relative amplitude in steps of 1/16 where each step effects a multiplier of 0.0625 so that step 16/16 effects a multiplier of 1.0. In such embodiments, a single shared 12-bit digital to analog converter (DAC) is further provided in order to deliver the master current, so that the respective 4 bit current sources all provide a scaled version of the master current from the 12 bit DAC. The master current is thus a single parameter which can be controlled by a clinician, by a patient or by a feedback loop. Thus, between the shared DAC which provides 12 bit control and the local DACs each providing a further 4 bits of control, 16 bits of control of each current source are provided of which 12 bits are in common and four bits are independent, with a majority of the control bits being in common.

In some examples, the related current sources can each be switchably connected to a selected one active stimulus electrode selected from a set of more than one available stimulus electrodes, to facilitate a device fitting process which includes selection of a subset of available electrodes for use for example to optimise dermatome selection or the like.

It is to be understood that neurostimulation requires a return path for delivered stimuli, and that a single return electrode may be shared between more than one active stimulus electrode. In alternative examples, each active stimulus electrode may be provided with an associated return or ground electrode. In such examples each return electrode may be provided with an associated return current source configured to effect return current steering. The return current sources may control each return electrode to return a respective return current which is defined in part by a shared return current control signal which is shared by each of the related return current sources, and which is defined in part by a respective unique return current control signal which is not shared by all of the related return current sources Moreover, the selection of return electrode(s) may be adaptive and/or configurable in order to further refine and control current steering and the perceived location of stimulation.

Additionally or alternatively, the or each return electrode may be connected directly to a power supply reference, such as a voltage supply rail. Such examples may be particularly advantageous in instances where it is desired to obtain a measurement of an ECAP evoked by the stimulus, as the stimulus artefact resulting from a known return electrode voltage can be predicted and compensated for, whereas the return electrode voltage is typically more difficult to predict and compensate for when a return current source is used and is imperfectly matched to the supply current source.

Still further examples may provide for a return current source to be provided for the or each return electrode, and may provide for a current value of the return current source to be controlled relative to the current value of the supply source so as to selectively define a resultant tissue voltage, so that a stimulus artefact resulting from a known return electrode voltage can be predicted and compensated for.

Examples of the present disclosure may thus be particularly useful in feedback control of neurostimulation, using ECAP measurements as the feedback variable. It is noted that a stimulation map is typically composed of a set of timing behaviours that specify aspects such as which electrodes are connected together during shorting and the order of events in the stimulation cycle, a set of parametric constants that specify aspects such as how long after each stimulus the first stage of an ECAP measurement amplifier should be blanked, and also a set of therapeutic variables that a clinician would normally change or set while programming a patient in order to optimise the selection of electrodes and the current steering between those electrodes and in order to define minimum and maximum stimulus levels and the like. Thus, applying feedback control to such a complex stimulation map is not always trivial, particularly given that stimulation occurs rapidly (e.g. in the tens or hundreds or thousands of Hz, or greater) and implant processing power, clock rates and battery capacity are limited which places significant constraints on feedback complexity. The solution of the present disclosure, of providing a shared current control signal which is shared by each of the related current sources, is thus particularly important in maintaining the ability to adjust a single parameter by feedback to effect a partial degree and preferably a large degree of common control over multiple current sources even as they continue to effect current steering.

According to a first aspect, the present invention provides an implantable neurostimulator comprising:
an implantable electrode array comprising at least one stimulus electrode and at least one return electrode, each electrode configured to deliver electrical stimuli to neural tissue;
an implantable control module configured to produce the electrical stimuli delivered by at least one stimulus electrode, the control module comprising at least one current injection current source configured to, in a first stimulus phase, pass a first current from a first supply rail to a stimulus electrode, and the control module further configured to connect the return electrode to a second supply rail during the first phase;
and the control module further comprising at least one current extraction current source configured to, in a second phase, pass a second current from a stimulus electrode to the second supply rail, and the control module further configured to connect the return electrode to the first supply rail during the second phase.

### Brief Description of the Drawings

An example of the invention will now be described with reference to the accompanying drawings, in which:
Figure 1 schematically illustrates an implanted spinal cord stimulator;
Figure 2 is a block diagram of the implanted neurostimulator;
Figure 3 is a schematic illustrating interaction of the implanted stimulator with a nerve;
Figure 4 is a detailed view of a plurality of current sources provided within the pulse generator of the present embodiment;
Figure 5A and 5B illustrate typical switch connections during stimulation, when using a normal passive ground return electrode (Fig. 5A) or when using a virtual ground configuration (Fig. 5B);
Figure 6 illustrates the control arrangement of each current source of Fig. 4;
Figure 7 provides a key to symbols and terminology;
Figures 8A to 8E show implementation of a virtual electrode; and
Figures 9A to 9E illustrate implementation of a virtual electrode, together with the use of a virtual ground, in accordance with another embodiment.

### Description of the Preferred Embodiments

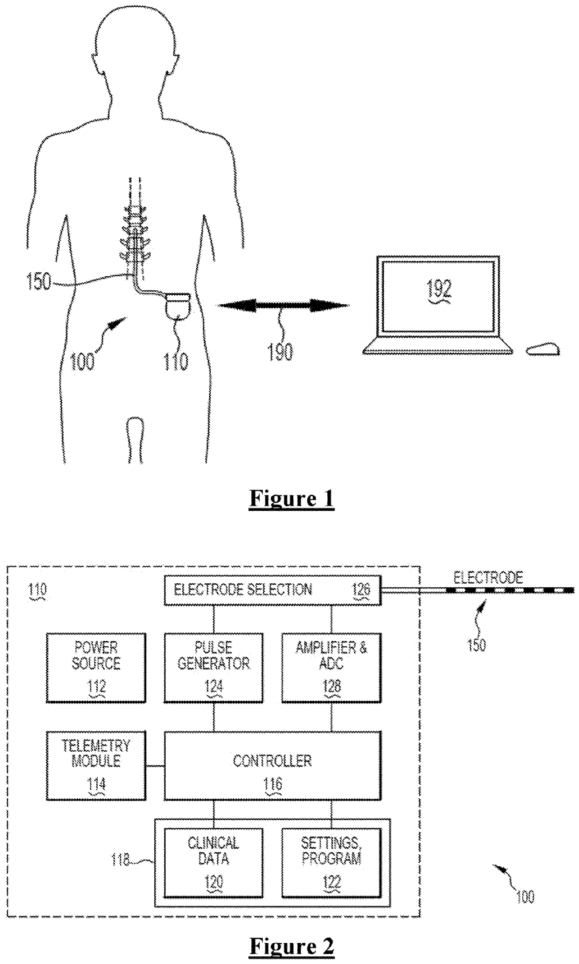

Figure 1 schematically illustrates an implanted spinal cord stimulator 100. Stimulator 100 comprises an electronics module 110 implanted at a suitable location in the patient's lower abdominal area or posterior superior gluteal region, and an electrode assembly 150 implanted within the epidural space and connected to the module 110 by a suitable lead. Numerous aspects of operation of implanted neural device 100 are reconfigurable by an external control device 192. Moreover, implanted neural device 100 serves a data gathering role, with gathered data being communicated to external device 192.

Figure 2 is a block diagram of the implanted neurostimulator 100. Module 110 contains a battery 112 and a telemetry module 114. In embodiments of the present invention, any suitable type of transcutaneous communication 190, such as infrared (IR), electromagnetic, capacitive and inductive transfer, may be used by telemetry module 114 to transfer power and/or data between an external device 192 and the electronics module 110.

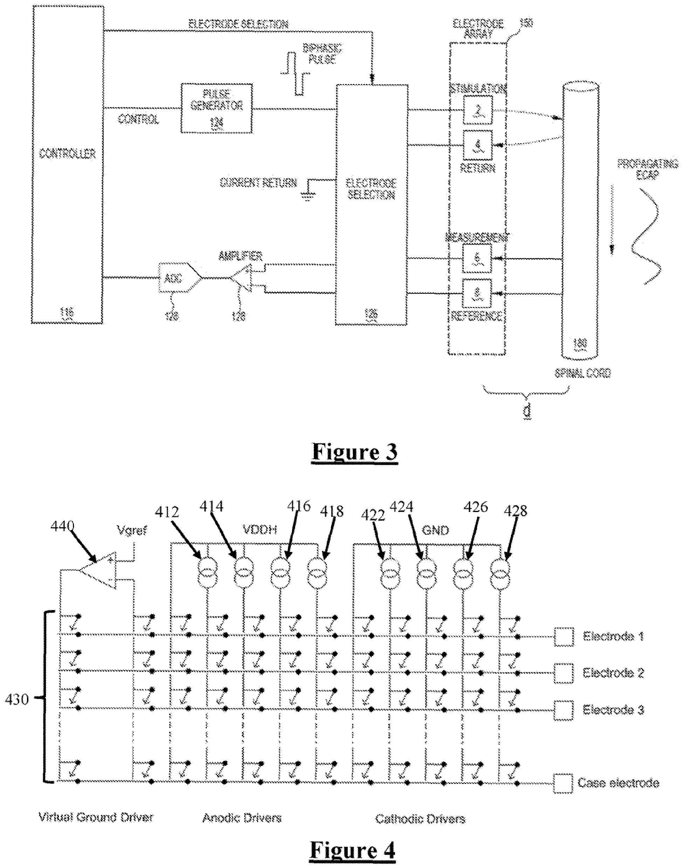

Module controller 116 has an associated memory 118 storing patient settings 120, control programs 122 and the like. Controller 116 controls a pulse generator 124 to generate stimuli in the form of current pulses in accordance with the patient settings 120 and control programs 122. Electrode selection module 126 switches the generated pulses to the appropriate electrode(s) of electrode array 150, for delivery of the current pulse to the tissue surrounding the selected electrode(s). For simplicity Figs 2 and 3 show a single pulse generator 124 delivering a bipolar stimulus via electrodes 2 and 4, however as such electrodes are typically positioned at about 7mm intervals the present invention further provides for current steering to be effected by use of additional pulse generators and additional stimulus electrodes, as described more fully in the following in relation to Figs 4 et seq. Measurement circuitry 128 is configured to capture measurements of neural responses sensed at sense electrode(s) of the electrode array as selected by electrode selection module 126.

Figure 3 is a schematic illustrating interaction of the implanted stimulator 100 with a nerve 180, in this case the spinal cord however alternative embodiments may be positioned adjacent any desired neural tissue including a peripheral nerve, visceral nerve, parasympathetic nerve or a brain structure. Electrode selection module 126 selects one or more stimulation electrode(s) 2 of electrode array 150 to deliver a triphasic electrical current pulse to surrounding tissue including nerve 180, although other embodiments may additionally or alternatively deliver a biphasic tripolar stimulus. Electrode selection module 126 also selects one or more return electrode(s) 4 of the array 150 for stimulus current recovery to maintain a zero net charge transfer.

Delivery of an appropriate stimulus to the nerve 180 evokes a neural response comprising a compound action potential which will propagate along the nerve 180 as illustrated, for therapeutic purposes which in the case of a spinal cord stimulator for chronic pain might be to create paraesthesia at a desired location. To this end the stimulus electrodes are used to deliver stimuli at 30 Hz. To fit the device, a clinician applies stimuli which produce a sensation that is experienced by the user as a paraesthesia. Current steering, described in more detail in the following, is used to identify an optimal location at which to apply such stimuli. When the paraesthesia is in a location and of a size which is congruent with the area of the user's body affected by pain, the clinician nominates that configuration for ongoing use.

The device 100 is further configured to sense the existence and electrical profile of compound action potentials (CAPs) propagating along nerve 180, whether such CAPs are evoked by the stimulus from electrodes 2 and 4, or otherwise evoked. To this end, any electrodes of the array 150 may be selected by the electrode selection module 126 to serve as measurement electrode 6 and measurement reference electrode 8. The stimulator case may also be used as a measurement electrode or reference electrode, or as a stimulation electrode or return electrode. Signals sensed by the measurement electrodes 6 and 8 are passed to measurement circuitry 128, which for example may operate in accordance with the teachings of International Patent Application Publication No. WO2012155183 by the present applicant, the content of which is incorporated herein by reference. The present invention recognises that in circumstances such as shown in Figure 3 where the recording electrodes are close to the site of stimulation, stimulus artefact presents a significant obstacle to obtaining accurate recordings of compound action potentials, but that reliable accurate CAP recordings are a key enabler for a range of neuromodulation techniques.

Fig. 4 provides a detailed view of a plurality of current sources provided within the pulse generator 124 and configured to effect current steering. As noted above, Figs 2 and 3 give a simplified view showing a single pulse generator 124 delivering a bipolar stimulus via electrodes 2 and 4. However as such electrodes are typically positioned at about 7mm intervals, the present invention further provides for current steering to be effected by use of additional pulse generators and additional stimulus electrodes, as shown in more detail in Fig. 4. Current sources 412, 414, 416, 418, also referred to as anodic drivers, are each referenced to a supply rail VDDH.

An additional set of return current sources 422, 424, 426, 428, also referred to as cathodic drivers, are referenced to a ground rail GND.

Current sources 412, 414, 416, 418, 422, 424, 426, 428 may each be selectably connected to any one respective electrode of the electrode array 150, by appropriately configuring each switch within the switch array 430 within electrode selection module 126.

In one embodiment the implant 100 has 24 epidural electrodes, of which only 3 are shown in Fig. 4, and one indifferent (case) electrode. The epidural electrodes are mounted on two electrode leads, with 12 electrodes on each lead, whereby the two leads are implanted alongside each other and substantially parallel to each other alongside the dorsal column, thus forming a 2 x 12 array of electrodes. The provision of four stimulus current sources 412, 414, 416, 418 and four current sources 422, 424, 426, 428 thus permits current steering to be effected in two dimensions, both axially (caudorostrally) along the lead between two selected stimulus electrodes, as well as laterally between the leads.

As further shown in Figure 4, a virtual ground amplifier 440 is also provided. Amplifier 440 can be selectably connected to any two respective electrodes of the electrode array 150, by appropriately configuring each switch within the switch array 430 within electrode selection module 126. Amplifier 440 operates in accordance with the teachings of International Patent Publication No. WO2014071445 by the present Applicant, the content of which is incorporated herein by reference.

Another aspect of the invention is illustrated in Figures 4-6. In particular, during a first phase of a stimulus the current sources 412-418 inject current to respective stimulus electrodes, while the return current sources 422-428 are disconnected, and a single return electrode is connected directly to the ground rail. Then, in a second phase of the stimulus, the return current sources 422-428 extract current from the stimulus electrodes in the same proportions as was injected in the first phase in order to ensure balanced net charge at each electrode. In this second phase the single return electrode is connected directly to the VDDH supply rail, and current sources 412-418 are disconnected. By positioning only one current source between the supply rails VDDH and GND in each phase, losses are reduced in this embodiment as compared to the use of a larger number of current sources. However, alternative embodiments may utilise one or more or all of the current sources 422-428 during the first phase, and/or may utilise one or more or all of current sources 412-418 during the second phase, for example to effect intra-phase charge balancing and/or to control the tissue voltage to a predictable value by current ratio control.

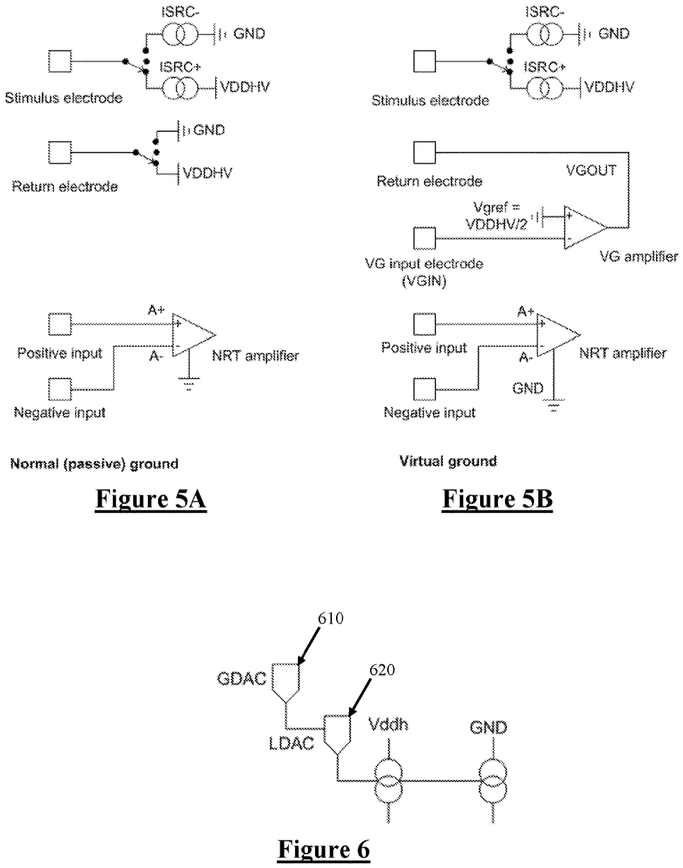

Figure 5A and 5B illustrate typical switch connections during stimulation, either when using a normal passive ground return electrode (Fig. 5A) or when using a virtual ground configuration (Fig. 5B). As will be understood, suitable switch sequences allow for delivery of a biphasic stimulus between each pair of stimulus electrodes, to ensure a zero net charge transfer and to also ensure that electrochemical effects at each electrode-tissue interface are reversed and/or generally to ensure that the stimulus regime meets safety requirements.

Figure 6 illustrates the control arrangement of each current source of Fig. 4, in accordance with the described embodiment. The current control consists of one global digital to analog converter (GDAC) 610, which controls four local DACs (LDACs) 620, of which only one is shown in Fig. 6.

The GDAC 610 has 12-bit control, of which 9 bits are monotonic. Each LDAC 620 has 4-bit control with 16 steps. There is one GDAC 610 for all four current source pairs, and four LDACs, one LDAC for each current source pair. The resolution of the LDAC is chosen as a small value, namely 4 bits, in order to simplify the design and cost. The present invention recognises that 4 bits of local control is adequate for field shaping, despite not being adequate for control of the current for general use. In this regard the currents from the electrodes therefore depend on the GDAC value to provide useful general function, such as for amplitude control in response to posture changes. The GDAC 610 and the LDAC multipliers go from 2⁻ⁿ to 1.0. The arrangement of Fig. 4 thus nominally has 18 bits of DAC control: 12 bits for the GDAC, 4 bits for each respective LDAC and 4 current sources (2 bits).

When there is a single stimulating electrode or fewer than four stimulating electrodes, either an individual current source may be used for the or each stimulating electrode, or multiple current sources may be used to drive a single stimulating electrode by switching each such current source output to that electrode's rail in the switch array 430. Such embodiments may be particularly advantageous in patients for whom the desired currents are high (6.25mA to 50mA), as in such cases it is preferable to use multiple current sources to reduce loss of compliance voltage. When currents are low, it is preferable to use a single current source. Tables 1 -4 below show the range of currents and recommended number of current sources in each case, in accordance with a preferred embodiment of the invention.

**Table 1: DAC Resolution - single stimulating electrode**

| | | | | |
|---|---|---|---|---|
| **Number of monotonic steps** | | | 512 | |
| **Resolution per current source (uA)** | | | 24.41 | |
| | | | | |

| **Patient's Maximum Current** | | | | |
|---|---|---|---|---|
| **Minimum (mA)** | **Maximum (mA)** | **Number of Current Sources** | **Resolution (uA)** | **Worst case step size** |
| 1.56 | 3.13 | 1 | 24.4 | 1.6% |
| 3.13 | 6.25 | 2 | 48.8 | 1.6% |
| 6.25 | 12.50 | 4 | 97.7 | 1.6% |
| 12.50 | 25.00 | 4 | 97.7 | 0.8% |
| 25.00 | 50.00 | 4 | 97.7 | 0.4% |

**Table 2: DAC Resolution - two stimulating electrodes**

| | | | | | |
|---|---|---|---|---|---|
| **Number of monotonic steps** | | | | 512 | |
| **Resolution per current source (uA)** | | | | 24.41 | |
| | | | | | |

| **Patient's Maximum Current** | | | | | |
|---|---|---|---|---|---|
| **Minimum (mA)** | **Maximum (mA)** | **Current src arrangement** | **Number of Current Sources** | **Resolution (uA)** | **Worst case step size** |
| 1.56 | 3.13 | 1+1 | 2 | 48.8 | 3.1% |
| 3.13 | 6.25 | 1+1 | 2 | 48.8 | 1.6% |
| 6.25 | 12.50 | 2+2 | 4 | 97.7 | 1.6% |
| 12.50 | 25.00 | 2+2 | 4 | 97.7 | 0.8% |
| 25.00 | 50.00 | 2+2 | 4 | 97.7 | 0.4% |

**Table 3: DAC Resolution - three stimulating electrodes**

| | | | | | |
|---|---|---|---|---|---|
| **Number of monotonic steps** | | | | 512 | |
| **Resolution per current source (uA)** | | | | 24.41 | |
| | | | | | |

| **Patient's Maximum Current** | | | | | |
|---|---|---|---|---|---|
| **Minimum (mA)** | **Maximum (mA)** | **Current src arrangement** | **Number of Current Sources** | **Resolution (uA)** | **Worst case step size** |
| 1.56 | 3.13 | 1+1+1 | 3 | 73.2 | 4.7% |
| 3.13 | 6.25 | 1+1+1 | 3 | 73.2 | 2.3% |
| 6.25 | 12.50 | 1+1+1 | 3 | 73.2 | 1.2% |
| 12.50 | 25.00 | 1+1+1 | 3 | 73.2 | 0.6% |
| 25.00 | 50.00 | 1+2+1 | 4 | 97.7 | 0.4% |

**Table 4: DAC Resolution - four stimulating electrodes**

| | | | | | |
|---|---|---|---|---|---|
| **Number of monotonic steps** | | | | 512 | |
| **Resolution per current source (uA)** | | | | 24.41 | |
| | | | | | |

| **Patient's Maximum Current** | | | | | |
|---|---|---|---|---|---|
| **Minimum (mA)** | **Maximum (mA)** | **Current src arrangement** | **Number of Current Sources** | **Resolution (uA)** | **Worst case step size** |
| 1.56 | 3.13 | 1+1+1+1 | 4 | 97.7 | 6.3% |
| 3.13 | 6.25 | 1+1+1+1 | 4 | 97.7 | 3.1% |
| 6.25 | 12.50 | 1+1+1+1 | 4 | 97.7 | 1.6% |
| 12.50 | 25.00 | 1+1+1+1 | 4 | 97.7 | 0.8% |
| 25.00 | 50.00 | 1+1+1+1 | 4 | 97.7 | 0.4% |

In accordance with the present invention, more than one stimulus electrode can be utilised to simultaneously deliver respective stimuli components, so that the plurality of stimulus electrodes collectively form what is referred to herein as a virtual electrode. The DACs are organized so that the implant can provide virtual electrodes of any arrangement permitted by configuring the switch array 430, with each such virtual electrode consisting of the combined effect of up to four independent stimulating electrodes. When creating a virtual electrode, the current delivered to each physical electrode can be selected to 4-bit accuracy by controlling the respective LDAC 620 for each electrode. The present invention recognises that 4 bit accuracy permits the virtual electrode to be selectively located at a virtual location which may be between the actual electrodes, and may be so located to a sufficient degree of accuracy under 4-bit control.

Moreover, by providing the GDAC in the described manner, the present invention advantageously also permits the GDAC to be used as a single feedback control variable in a feedback loop. The feedback loop may be based on measurements of evoked compound action potentials, and may for example operate in accordance with the teachings of one or more of International Patent Publication WO2012155188, International Patent Publication WO2016090436 and International Patent Publication WO2017173493, by the present Applicant, the contents of each being incorporated herein by reference. In such a feedback loop, the controlled variable drives the global DAC 610 and the local DACs can each remain static, significantly simplifying the implementation of such a feedback loop.

The implant fitting process, as for example may be saved into firmware, should specify a single LDAC setting as part of a therapy map suitable for the patient concerned.

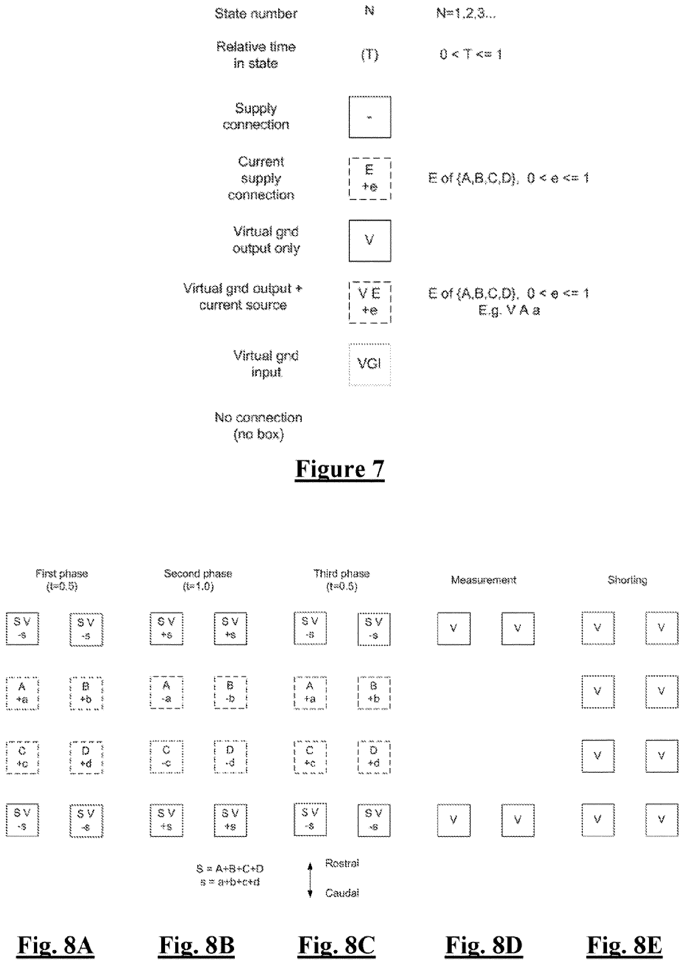

Figures 7 to 9 further illustrate the spatiotemporal nature of stimulation options made possible by the present invention.

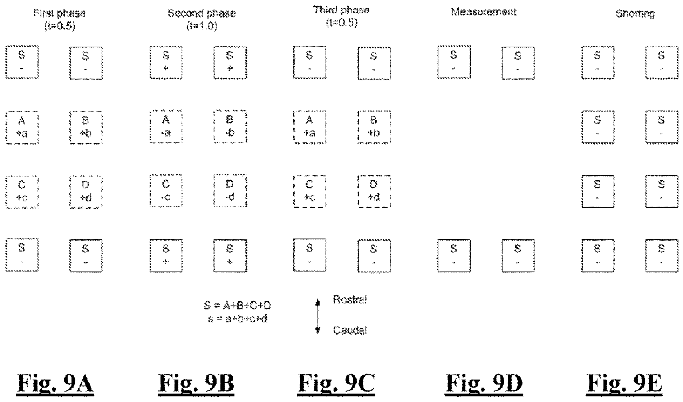

Figure 7 provides a key to the symbols and terminology utilised in Figs 8 & 9. These diagrams show a 4x2 array (or a 4x2 portion of an array) of electrodes. An electrode connected to a supply is shown with a solid line, with the sign of the supply written inside. Current sources are shown with a sign and the proportion of current borne by that respective electrode. When more than one current source is used, individual units are marked with the letter (A-D). Stimulus phases are numbered 1... n, measurement and charge recovery phases are marked "M" and "C". Time travels from left to right, whereby a first stimulus phase is portrayed in Fig. 8A, a second stimulus phase is portrayed in Fig. 8B, a third stimulus phase is portrayed in Fig. 8D, a measurement phase is portrayed in Fig. 8D and a shorting phase is portrayed in Fig. 8E. The stimulus electrode is usually cathodic, with other electrodes referred to as the anodic or return electrode. The values a,b,c,d are LDAC values to effect a virtual electrode, and are set for the patient's map as required for a desired therapeutic outcome.

Figures 8A to 8E thus show implementation of a virtual electrode interposed between actual electrodes A-D, in a tri-phasic stimulation configuration (Figs 8A-8C), followed by a measurement phase (Fig. 8D) and finally an electrode shorting phase (Fig. 8E).

Figures 9A-9E illustrate implementation of a virtual electrode, together with the use of a virtual ground in accordance with WO2014071445 noted previously herein, in a tri-phasic stimulation arrangement.

The GDAC value is a single value for each stimulus. Upon completion of each measurement phase (e.g Fig 8D, 9D) the GDAC value can be changed via feedback based on the measurement result, to thus effect feedback controlled neural stimulation via a virtual electrode.

It will be appreciated by persons skilled in the art that numerous variations and/or modifications may be made to the invention as shown in the specific embodiments without departing the invention as broadly described and as defined in the appended claims.

## Claims

1. An implantable neurostimulator (100) comprising:
an implantable electrode array (150) comprising at least one stimulus electrode and at least one return electrode, each electrode configured to deliver electrical stimuli to neural tissue;
an implantable control module (110) configured to produce the electrical stimuli delivered by the at least one stimulus electrode, **characterised in that** the control module comprises at least one current injection current source configured to, in a first stimulus phase, pass a first current from a first supply rail to a stimulus electrode, and the control module further configured to connect the at least one return electrode to a second supply rail during the first stimulus phase;
and the control module further comprising at least one current extraction current source configured to, in a second stimulus phase, pass a second current from a stimulus electrode to the second supply rail, and the control module further configured to connect the at least one return electrode to the first supply rail during the second stimulus phase.

2. The implantable neurostimulator (100) of claim 1, wherein the second supply rail is a ground rail.

3. The implantable neurostimulator (100) of claim 1 or claim 2, wherein the first supply rail is a voltage supply rail.

## Patentansprüche

1. Implantierbarer Neurostimulator (100), umfassend:
eine implantierbare Elektrodenanordnung (150), umfassend wenigstens eine Stimulationselektrode und wenigstens eine Gegenelektrode, wobei jede Elektrode konfiguriert ist, um elektrische Reize an Nervengewebe abzugeben;
ein implantierbares Steuermodul (110), das konfiguriert ist, um die elektrischen Reize, die von der wenigstens einen Stimulationselektrode abgegeben werden, zu erzeugen, **dadurch gekennzeichnet, dass** das Steuermodul wenigstens eine Strominjektions-Stromquelle umfasst, die konfiguriert ist, um in einer ersten Stimulationsphase einen ersten Strom von einer ersten Versorgungsschiene an eine Stimulationselektrode zu leiten, und das Steuermodul ferner konfiguriert ist, um die wenigstens eine Gegenelektrode mit einer zweiten Versorgungsschiene während der ersten Stimulationsphase zu verbinden;
und wobei das Steuermodul ferner wenigstens eine Stromextraktions-Stromquelle umfasst, die konfiguriert ist, um in einer zweiten Stimulationsphase einen zweiten Strom von einer Stimulationselektrode an die zweite Versorgungsschiene zu leiten, und das Steuermodul ferner konfiguriert ist, um die wenigstens eine Gegenelektrode mit der ersten Versorgungschiene während der zweiten Stimulationsphase zu verbinden.

2. Implantierbarer Neurostimulator (100) nach Anspruch 1, wobei die zweite Versorgungschiene eine Erdungsschiene ist.

3. Implantierbarer Neurostimulator (100) nach Anspruch 1 oder Anspruch 2, wobei die erste Versorgungsschiene eine Spannungsversorgungsschiene ist.

## Revendications

1. Un neurostimulateur implantable (100) comprenant :
une matrice d'électrodes implantable (150) comprenant au moins une électrode de stimulation et au moins une électrode de retour, chaque électrode étant configurée pour délivrer des stimuli électriques aux tissus neuronaux ;
un module de contrôle implantable (110) configuré pour produire les stimuli électriques délivrés par la ou les électrodes de stimulation, **caractérisé en ce que** le module de contrôle comprend au moins une source de courant d'injection configurée pour, dans une première phase de stimulation, faire passer un premier courant d'un premier rail d'alimentation vers une électrode de stimulation, et le module de contrôle est en outre configuré pour connecter l'électrode de retour au second rail d'alimentation durant la première phase de stimulation ;
et le module de contrôle comprenant en outre au moins une source de courant d'extraction configurée pour, dans une seconde phase de stimulation, faire passer un second courant d'une électrode de stimulation vers le second rail d'alimentation, et le module de contrôle est en outre configuré pour connecter l'électrode de retour au premier rail d'alimentation durant la seconde phase de stimulation.

2. Le neurostimulateur implantable (100) selon la revendication 1, dans lequel le second rail d'alimentation est un rail de masse.

3. Le neurostimulateur implantable (100) selon la revendication 1 ou la revendication 2, dans lequel le premier rail d'alimentation est un rail d'alimentation en tension.
